# FASCICULE DE BREVET EUROPEEN

(11) **EP 0 806 185 B1**
(45) Date de publication et mention de la délivrance du brevet: **27.11.2002**
(21) Numéro de dépôt: 97810280.4
(22) Date de dépôt: 05.05.1997
(51) Int. Cl.: A61B 17/60

(54) **Fixateur externe**
Externe Fixationsvorrichtung
External fixator

(30) Priorité: 10.05.1996 CH 120696
(43) Date de publication de la demande: 12.11.1997
(73) Titulaire: Stryker Trauma S.A., 2545 Selzach (CH)
(72) Inventeur: Mata, Jacques, 1163 Etoy (CH); Nyfeler, Marcel, 1163 Etoy (CH); Worek, Denis, 1255 Veyrier (CH)
(74) Mandataire: Dietlin, Henri

(56) Documents cités:
- EP-A- 0 385 929
- EP-A- 0 700 664
- DE-A- 3 614 305
- FR-A- 2 405 063
- FR-A- 2 616 059

## Description

La présente invention est du domaine de l'orthopédie et concerne plus particulièrement un fixateur externe destiné à la consolidation d'un os long, fracturé vers son extrémité.

Depuis de nombreuses années, on a développé des fixateurs externes dont les composants sont mis en place après que les fiches aient été insérées dans la position optimale par rapport au fragment osseux à maintenir et au tissu l'entourant. Des articulations assurent la liaison entre les fiches et la ou les barre(s) de rigidification, pour assurer leur orientation relative. La titulaire a plus particulièrement développé à cette fin l'articulation décrite dans la demande de brevet européen No 0 700 664 qui permet d'orienter soit une fiche et une barre, soit deux barres entre elles. Cette articulation comporte plusieurs paires de mâchoires formant des rainures positionnées et agencées de manière à présenter une ouverture extérieure permettant le clipsage de la pièce cylindrique par pression de cette dernière à l'encontre des moyens élastiques que pressent les faces adjacentes des mâchoires l'une contre l'autre, pour maintenir l'articulation sur les pièces cylindriques avant le blocage de l'articulation.

Dans certains cas, on est amené à utiliser un fixateur comportant une barre de rigidification unique et non pas une série de barres formant un cadre de rigidification. Pour ce type de fixateur, des paires d'au moins deux fiches osseuses sont insérées de part et d'autre de la fracture et sont indirectement fixées aux extrémités d'une barre de rigidification. Lorsqu'un os long est fracturé au voisinage d'une articulation, il est préférable d'insérer les fiches dans une disposition dite en « T », certaines des fiches étant insérées dans l'épiphyse dans un plan perpendiculaire à l'os, les autres étant insérées dans la partie centrale de l'os, dans un plan contenant l'os fracturé. Chaque groupe de fiches est maintenu dans un étau que l'ont peut orienter par rapport à la barre de rigidification grâce à une articulation ou une rotule.

Le titulaire a également développé un fixateur externe décrit dans le brevet européen No 385 929. Le fixateur, qui est un modèle spécialement destiné a régler la force appliquée aux fragments osseux pour obtenir des allongements ou des compressions est extrêmement lourd et complexe. De plus, il ne peut être utilisé dans le cas où les groupes de fiches sont parallèles, la possibilité de rotation des mâchoires par rapport à l'axe des fiches étant trop limitée.

Il existe également un fixateur décrit dans la demande allemande DOS No 36 14 305 permettant de placer des groupes de fiches perpendiculairement l'un par rapport à l'autre. Ce fixateur présente cependant des mâchoires fixes à un seul degré de liberté.

La présente invention vise à simplifier ce type de montage et à diminuer l'encombrement et le poids de l'ensemble, plus particulièrement étudié pour les os longs, tels le radius, fracturés aux environs de leur extrémité. Elle a pour objet un fixateur comportant au moins :
- deux groupes de fiches insérées respectivement de part et d'autre de la fracture,
- deux étaux de fixation des fiches,
- deux articulations assurant l'orientation des étaux par rapport à une barre de rigidification.

Elle est caractérisée par le fait que l'une des mâchoires de chaque étau comporte une barrette cylindrique destinée à coopérer avec une articulation, ladite barrette étant parallèle au plan des fiches et perpendiculaire par rapport à l'axe de celle-ci.

Le dessin annexé représente à titre d'exemple non limitatif une forme d'exécution de l'objet de la présente invention.

La figure 1 représente l'étau selon l'invention, vu de face dans la partie gauche du dessin et en coupe dans la partie droite.

La figure 2 représente l'étau de la figure 1 vu latéralement et représenté monté sur une articulation du type de celle décrite dans la demande de brevet européen No 0 700 664 au nom de la titulaire.

La figure 3 est une vue en perspective de l'ensemble du fixateur selon l'invention.

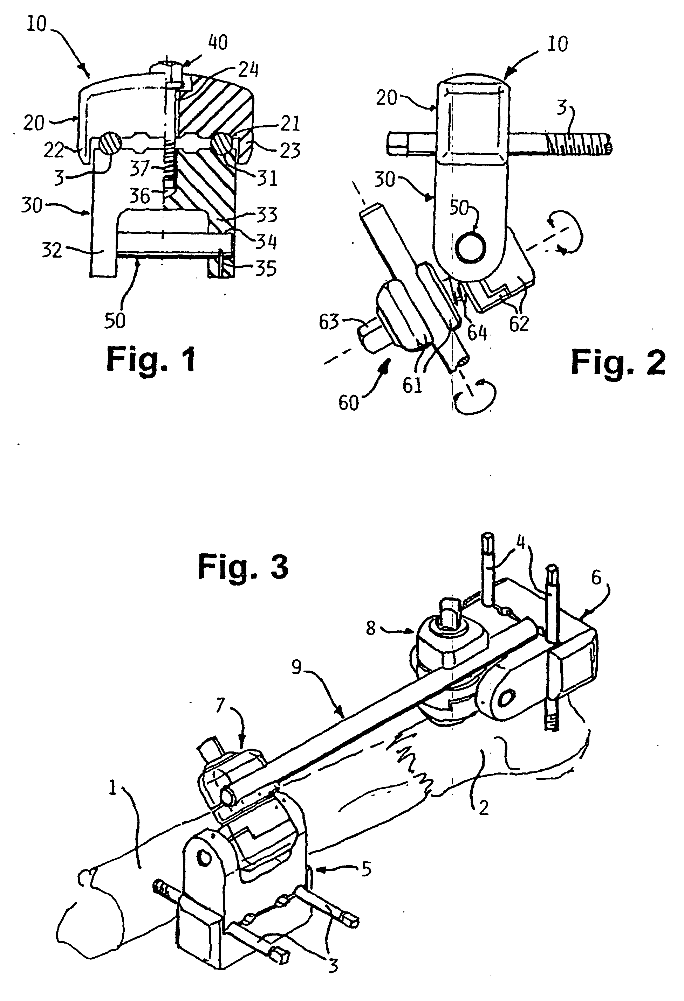

L'étau 10 représenté à la figure 1 est constitué de manière conventionnelle par une mâchoire supérieure 20 et une mâchoire inférieure 30 reliées par une vis centrale de serrage 40. Ces mâchoires 20 et 30 comportent des séries de rainures parallèles 21 et 31 pour les fiches osseuses 3.

La mâchoire supérieure 20 comporte en outre deux ailes 22 et 23 de positionnement relatif des mâchoires et un passage central 24 dans lequel la vis de serrage 40 passe librement.

La mâchoire inférieure 30 a une forme générale de "U" (à l'envers au dessin), dont les jambages 32 et 33 sont destinés à recevoir une barrette 50 dont les extrémités sont insérées dans un passage 34 pratiqué dans chaque jambage, et fixées au moyen d'une goupille 35 destinée à empêcher toute rotation de la barrette. La mâchoire inférieure 30 comporte également un passage central 36 destiné à coopérer avec la vis 40. De préférence un manchon métallique fileté 37 est intercalé entre le passage 36 et la vis 40. Par construction la barrette 50 est parallèle au plan contenant les fiches et, comme visible au dessin, elle est perpendiculaire à l'axe des fiches osseuses.

Dans la vue de côté de la figure 2 on retrouve les mâchoires 20 et 30 formant l'étau 10 de serrage de fiches 3, la mâchoire inférieure 30 présentant la barrette 50 destinée à la fixation d'une articulation 60. Celle-ci comporte une première paire de mâchoires 61 serrées sur une barre d'écartement et une seconde paire de mâchoires 62 serrées sur la barrette 50, ces paires de mâchoires pouvant être orientées par rapport à un axe central constitué par la vis de serrage 63 qui constitue l'axe de rotation des mâchoires. On peut prévoir de séparer les paires de mâchoires 61 et 62 par un ressort hélicoïdal 64, destiné à tenir les composants en place avant que la vis 63 soit totalement serrée, comme détaillé dans la demande de brevet européen No 0.700.664 déjà citée.

Dans la vue générale de la figure 3 on a représenté un os long 1 fracturé relativement près de l'extrémité distale 2 du radius, région dans laquelle ont lieu plus de 20% des fractures des membres. Chaque fragment osseux 1 ou 2 reçoit respectivement au moins deux fiches 3 ou 4, disposées de préférence en "T". Ces fiches sont serrées dans deux étaux 5 et 6 coopérant avec des articulations 7 et 8 entre lesquelles est disposée une barre 9 de rigidification. Le médecin pourra réduire la fracture et remettre les fragments osseux 1 et 2 dans leur position optimale grâce à la combinaison des mouvements de rotation :
- des articulations 7 et 8 par rapport à l'axe de la barre 9,
- des paires de mâchoires de chaque articulation 7 ou 8 sur leur axe de serrage 63,
- des articulations 7 et 8 par rapport à la barrette de chaque étau 5 ou 6.

Dans la représentation de la figure 3 les fiches 3 sont dans un plan horizontal tandis que les fiches 4 sont dans un plan vertical. On notera que l'axe de l'articulation 8 est sensiblement parallèle aux fiches 4, tandis que l'axe de l'articulation 7 est à 45° du plan contenant les fiches 3.

Dans les fractures du poignet, on a remarqué que l'on a avantage à disposer de préférence les fiches 3 dans un plan à 45° de l'horizontale, l'axe de l'articulation 7 étant sensiblement aligné sur l'axe central de serrage de l'étau 5. Selon la région d'utilisation du fixateur selon l'invention, on peut prévoir de disposer les deux groupes de fiches dans des plans sensiblement parallèles, et non dans la disposition en "T" telle que décrite précédemment, sans sortir du cadre de la présente invention.

Le procédé de mise en place du fixateur décrit jusqu'ici est consiste à insérer des paires de fiches dans les fragments osseux de part et d'autre de la fracture, à serrer les fiches dans les étaux, à monter les articulations sur les étaux et sur une barre de rigidification, avant de procéder au réglage final de la position des fragments osseux grâce à la combinaison des mouvements de rotation des articulations ou en d'autres termes le positionnement de la barre de rigidification par rapport aux fiches.

Il est à noter encore que l'on utilisera avantageusement des étaux et même une barre de rigidification en matériaux composites transparents aux rayons X pour vérifier aisément le positionnement relatif des fragments osseux lors de la mise en place du fixateur. Par exemple on utilisera des matières plastiques renforcées, de préférence à base de fibres de carbone.

De préférence, les barrettes 50 et la barre d'écartement 9 ont le même diamètre, ce qui permet une plus grande liberté dans le positionnement des composants et de limiter au maximum l'encombrement et les parties en saillie des mâchoires et des articulations, tout en autorisant un accès facile aux pièces de serrage.

## Revendications

1. Fixateur externe comportant au moins
• deux groupes de fiches (3,4) insérées respectivement de part et d'autre de la fracture,
• deux étaux (5, 6 ; 10) de fixation des fiches,
• deux articulations (7,8 ; 60) assurant l'orientation des étaux par rapport à une barre de rigidification (9),
**caractérisé par le fait que** l'une des mâchoires de chaque étau comporte une barrette cylindrique (50) destinée à coopérer avec une articulation, ladite barrette (50) étant parallèle au plan des fiches (3) et perpendiculaire par rapport à l'axe de celles-ci.

2. Fixateur selon la revendication 1, **caractérisé par le fait que** chaque étau comporte une mâchoire (30) en forme de U dont les jambages (32, 33) comportent deux ouvertures (34) vis-à-vis, dans lesquelles sont insérées les extrémités de la barrette (50).

3. Fixateur selon la revendication 2, **caractérisé par le fait que** ledit jambage (32, 33) comporte en outre un passage pour un moyen de blocage (35) de la barrette (50) en rotation.

4. Fixateur selon la revendication 1 **caractérisé par le fait que** les mâchoires 20 et 30 de l'étau sont en matériaux plastiques renforcés.

## Patentansprüche

1. Externer Fixateur, der mindestens folgendes aufweist:
* zwei Stiftgruppen (3, 4), respektive beiderseits der Fraktur eingefügt,
* zwei Schraubstöcke (5, 6; 10) zum Einspannen der Stifte,
* zwei Gelenke (7, 8; 60) zur Ausrichtung der Schraubstöcke in bezug auf eine Festigungsstange (9),
**dadurch gekennzeichnet, dass** eine der Klemmbacken jedes Schraubstocks einen Zylindersteg (50) enthält, der zur Zusammenarbeit mit einem Gelenk bestimmt ist, wobei der besagte Steg (50) parallel zur Stiftfläche (3) und rechtwinklig in bezug auf deren Achse angeordnet ist.

2. Fixateur nach Anspruch 1, **dadurch gekennzeichnet, dass** jeder Schraubstock eine Klemmbacke (30) in U-Form aufweist, deren Unterteile (32, 33) zwei gegenüberliegende Öffnungen (34) aufweisen, in die die Enden des Stegs (50) eingefügt werden.

3. Fixateur nach Anspruch 2, **dadurch gekennzeichnet, dass** das besagte Unterteil (32, 33) ausserdem eine Öffnung für eine Sperrvorrichtung (35) des Stegs (50) gegen Drehungen aufweist.

4. Fixateur nach Anspruch 1, **dadurch gekennzeichnet, dass** die Klemmbacken (20, 30) des Schraubstocks aus verstärktem Kunststoffmaterial sind.

## Claims

1. External fixator with at least:
• two sets of pins (3, 4) respectively inserted on each side of the fracture,
• two pin clamps (5, 6; 10),
• two hinges (7, 8; 60) allowing for the orientation of the clamps relative to a stiffening rod (9),
**characterised by** the fact that one of the jaws on each clamp has a cylindrical crossbar (50) designed to work in conjunction with a hinge, the aforementioned crossbar (50) being parallel to the plane of the pins (3) and perpendicular relative to their axis.

2. Fixator in accordance with claim 1, **characterised by** the fact that each clamp has a U-shaped jaw (30), the jambs (32, 33) of which have openings (34) opposite each other in which the ends of the crossbar (50) are inserted.

3. Fixator in accordance with claim 2, **characterised by** the fact that the aforementioned jamb (32, 33) also has a passage for a locking pin (35) to fix the crossbar (50) to prevent rotation.

4. Fixator in accordance with claim 1 **characterised by** the fact that the jaws 20 and 30 of the clamp are made of reinforced plastic.
